# EUROPEAN PATENT APPLICATION

(11) **EP 2 749 557 A1**
(43) Date of publication of application: **02.07.2014**
(21) Application number: 12199793.6
(22) Date of filing: 31.12.2012
(51) Int. Cl.: C07D 401/04

(54) **Process for preparation of alpha polymorph of imatinib mesylate from IPA and THF solvate forms of imatinib mesylate**

(71) Applicant: Deva Holding Anonim Sirketi, 34303 Istanbul (TR)
(72) Inventor: Koc, Fikret, 34303 ISTANBUL (TR); Karliga, Bekir, 34303 ISTANBUL (TR); Bellur Atici, Esen, 34303 ISTANBUL (TR); Ilhan, Suna Ayse, 34303 ISTANBUL (TR); Yilmaz, Esra Mujde, 34303 Istanbul (TR)

(57) **Abstract**

The present invention relates to a process for preparation of imatinib mesylate alpha crystal form b desolvation of pseudo-polymorphic solvate forms of imatinib mesylate.

## Description

### Technical Field

The present invention relates to a process for preparation of imatinib mesylate α crystal form and mixture of crystal forms α and β of imatinib mesylate from pseudo-polymorphic solvate forms of imatinib mesylate.

### Background Art

Imatinib mesylate, 4-(4-methylpiperazin-1ylmethyl)-N (4-methyl-3-[4-pyrinin-3-yl) pyrimidin-2-yloamino] phenyl] benzamide mesylate, has a molecular formula of C₂₉H₃N₇O.CH₄SO₃ and a molecular weight of 589.7.

WO 95/09852 A (CIBA GEIGY AG) 13.04.1995 discloses firstly the preparation of imatinib as free base.

Imatinib is the first member of a new class of agents that act specially by inhibiting a certain enzyme that is characteristic of a particular cancer cell.

Imatinib is a protein-tyrosine kinase inhibitor, especially useful in the treatment of various types of cancer and can also be used for the treatment of atherosclerosis, thrombosis, restenosis, or fibrosis. Thus, imatinib can be also used for the treatment of non-malignant diseases.

Imatinib is usually administered orally in the form of a suitable salt, e.g., in the form of imatinib mesylate. The recommended dosage of imatinib is 400 mg per day for patients in chronic phase CML and 600 mg per day for patients in accelerated phase or blast crisis.

Recently, in solid state pharmaceutical science, development of new crystal forms of active pharmaceutical ingredients is an ongoing challenge and it is a routine practice to investigate in development for new candidates of polymorphism for active pharmaceutical ingredients.

It is well known that imatinib mesylate may crystallize in different polymorphic forms.

WO 99/03854 A (NOVARTIS-ERFINDUNGEN VERWALTUNGSGESSELLSCHAFT MBH) 28.01.1999 discloses two crystalline α crystalline and β crystalline forms of imatinib mesylate and preparation thereof.

WO 2004/106326 A (HETERO DRUGS LIMITED) 09.12.2004 discloses firstly a hydrate form of imatinib mesylate and preparation thereof.

Moreover, there were shown many examples regarding to preparation of imatinib mesylate polymorphic form α in the literature.

WO 2009/151899 A (DR. REDDY'S LABORATORIES) 17.12.2009 describes a method of preparing of imatinib mesylate solvate which involves the use of ether solvent, such as cyclic ethers (tetrahydrofuran, pentahydropyran, and the like) and acyclic ethers.

US 20090264438 (JEGOROV CHUDIK ARONHIME) 22.10.2009 describes a preparation of imatinib mesylate solvate by a method of preparation of a solvent selected from the group consisting of: ether, tetrahydrofuran, dioxolane, aliphatic alcohol, nitromethane and acetic acid.

WO 2005/095379 A (INSTYTUT FARMACEUTYCZNY) 13.10.2005 discloses a process to prepare imatinib mesylate which is carried out in the mixture isopropanol and aliphatic alcohols, by yielding formation of α polymorphic form.

WO 2006/048890 A (SUN PHARMACEUTICAL INDUSTRIES LIMITED) 11.05.2006 describes a process for non-needle shaped alpha-crystalline from of imatinib mesylate which includes subjecting a solution of imatinib mesylate in a suitable solvent (which may be a polar protic or aprotic solvent, a non-polar solvent, water or mixture thereof) to be agitated thin film drying under atmospheric pressure and/or under vacuum.

US 7638627 (CIPLA LIMITED) 29.12.2009 discloses a method of preparing α crystalline form imatinib mesylate which involves suspending of imatinib base in isopropanol, adding methane sulphonic acid, heating the solution to reflux and concentrating the reaction mixture to a minimum volume, cooling the reaction mixture and isolating the alpha-crystalline form.

All above mentioned patents disclose processes including crystallisation of imatinib mesylate by isolation in isopropanol or tetrahydrofuran as chosen solvent. In spite of most of those processes contain drying step required to remove excess of isopropanol and tetrahydrofuran as organic residual volatile, a complete removal of said solvents can be difficult. Therefore, there is a need for new processes which are applicable in industrial scale and being economical.

### Summary of invention

The present invention relates to preparation of pure crystalline form of imatinib mesylate. More specifically, the invention relates to preparation of pure crystalline alpha form of imatinib mesylate by desolvation of IPA and THF pseudo-polymorphic solvate forms of imatinib mesylate.

The present invention also relates to preparation of pure polymorphic form alpha of imatinib mesylate from the mixture of alpha and beta crystalline forms of imatinib mesylate via desolvation of IPA and THF pseudo-polymorphic solvate forms of imatinib mesylate.

### Technical Problem

Recently, pharmaceutical manufacturing processes and formulations are regulated according to some specific guidelines, which are issued from authorities such as the International Conference on Harmonization (ICH), the United States Food and Drug Administration (US FDA), the European Medicines Agency (EMA), the Canadian Drug and Health Agency, the Japanese Pharmaceutical and Medical Devices Agency (PMDA), and the Australian Department of Health and Ageing Therapeutic Goods.

These guidelines consist of appropriate specifications in accordance with accepted standards and consistent with the manufacturing process. The specifications include control of impurities (e.g., organic impurities, inorganic impurities, and residual solvents) and their appropriate limits.

In addition, a number of official compendia, such as the British Pharmacopoeia (BP), the United States Pharmacopeia (USP), the Japanese Pharmacopoeia (JP), and the European Pharmacopoeia (EP) are setting standards limits to restrict the impurity levels existent in APIs as well as in finished products.

In pharmaceutical technology, the removal of residual solvent has received much scrutiny throughout various stages of drug development due to their toxic, physiologic or pharmacological effects.

It is quietly common that final purification step of most pharmaceutical drug substance processes involves a crystallization step in which solvent molecules may become entrapped or bound within the crystal lattice when crystals of a compound are forming.

The occurrence of a compound crystallizing in different three dimensional lattices based upon the presence of solvent molecules is named as pseudo-polymorphism, also known as solvate. But formation of solvates which can act as residual impurity is not desirable by manufacturers.

Crystal solvates and pseudo-polymorphic solvates are well defined in the literature.

As used herein, the term "solvates" refers to pseudo-polymorphic solvates in this invention. Pseudo-polymorphic solvates are considered as residual solvents. Therefore, it would be necessary to desolvate pseudo-polymorphic solvates to meet pharmaceutically accepted levels of residual solvent content.

Several methods are presently known and used in industry for reducing the residual content of solvent. Among them, compound treated with vacuum drying to promote evaporation of residual solvent may be given as an example. Nevertheless, excessive heating during drying may cause degradation of product or impact significantly the properties, functionality of the product.

Therefore, a more economical desolvation process is always needed in the pharmaceutical industry.

Similarly, many processes in which solvents like isopropanol or tetrahydrofuran are used, solvation usually occurs during purification step or isolation step of imatinib. However, the lengthy time of vacuum drying by recrystallization of the solvated imatinib mesylate from a suitable solvent remains as a problem.

As a result, in view of the known methods associated with the manufacturing of pure polymorphic imatinib mesylate, there is a constant need to develop easy, economically preferred, reliable process to remove residual solvents without heating at high temperatures for a long period of time.

The present invention is directed to overcome these and other problems encountered in the art.

### Solution to Problem

In the present invention, it is aimed to develop an easy to produce desolvation process to produce imatinib mesylate which contains residual solvent within acceptable levels.

Inventors have developed an improved method and shortened time required for drying period in the manufacturing of pure polymorphic imatinib mesylate.

As used herein, unless otherwise indicated, "imatinib mesylate" includes alpha polymorphic form.

In contrast to known desolvation of imatinib-IPA (or THF) solvates via drying at high temperatures for a long time, the present technique comprises desolvation of said solvates by additional recrystallization step.

Accordingly, non-bounded isopropyl alcohol (IPA) or tetrahydrofuran (THF) in the crystal lattice of imatinib mesylate is removed by re-crystallization with a second more volatile solvent system (or mixture) like mixture of diisopropyl ether and isopropyl alcohol.

The process described in the present invention involves following steps of seeding with an alpha crystal form of imatinib mesylate-IPA solvate and desolvation with use of solvent mixture IPA and diisopropyl ether.

Inventors have surprisingly found that recrystallization of imatinib solvates from a mixture of IPA: DIPE by seeding with crystals of imatinib mesylate has considerably reduced the drying time.

In another aspect, of this invention, the provided method is also applicable for preparation of alpha-crystalline form of imatinib mesylate from the mixture of alpha and beta crystalline forms of imatinib mesylate.

By desolvation process in presented invention, the removal of solvent under mild conditions meets the criteria for ICH requirements (International Conference on Harmonisation of Technical Requirements for Registration of Pharmaceuticals for Human Use), which regard pharmaceutically accepted level of residual solvents assay of pharmaceuticals for human use. In ICH of Technical Requirements for Registration of Pharmaceuticals for Residual Solvents (Q3C (R5) dated February 2012), the content of residual solvent is determined as follows; below 720 ppm for THF and below 5000 ppm for IPA.

The said method of desolvation avoids extended period of drying under vacuum and greatly decreases the cost in the synthesis of imatinib mesylate and it is propitious to industrialized production.

The process for preparation of pure imatinib mesylate from IPA- solvated form or THF-solvated form is in a reproducible manner.

Additional advantages will be set forth in the description that follows.

### Brief description of drawings

Fig.1 provides the scanning electron microscope image of the α-crystal form of imatinib mesylate solvated with IPA according to the process of the present invention.

Fig.2 provides the scanning electron microscope image of the α-crystal form of imatinib mesylate solvated with THF according to the process of the present invention.

Fig.3 provides the scanning electron microscope image of the α-crystal form of imatinib mesylate after desolvation of IPA or THF-imatinib mesylate solvate according to the process of the present invention.

### Description of embodiments

Chemical stability, solid state stability and shelf-life are important properties of pharmaceutical active compounds. It is generally known that stability of the pharmaceutical ingredients depends also upon residual solvents.

Residual solvents may be present in drug substances, excipients, and in drug products. Production or purification processes are known to result in the presence of residual solvents. Most of practical manufacturing techniques when crystallization may end up with formation of solvates.

Solvates can be defined as two types, namely "crystal solvate" and "pseudo-polymorphic solvate". Crystals that contain solvent of crystallisation are called crystal solvates. Crystal solvate means that they may entrap solvent when some compounds crystallise in the crystal. Another type of solvate is pseudo-polymorphic solvate, meaning that, the solvent is not a part of the crystal bonding and merely occupies voids in the crystal. Desolvation does not destroy the crystal lattice.

Although ICH guidelines define acceptable levels for residual solvents in pharmaceuticals, it does not address solvates. Additionally, it is stated that desolvation of solvates leads to elevated levels of residual solvent. So, the solvent content of pharmaceutical products should be evaluated and justified. The residual solvent levels in the drug product should be equal or below to the recommended level in the guidelines. If the measured level of residual solvent is above the recommended level, manufacturing method of drug product should be adopted to reduce the relevant solvent level to within the acceptable amount.

Therefore, it is highly desirable to provide stable and crystalline solid form of the pharmaceutical ingredient with low level of residual solvents in an industrially preferable, simple and reproducible process.

The removal of undesirable residual solvent is generally achieved by heating and/or by applying a vacuum. A wide variety of dryers are available on the market and the selection of optimal drying equipment is performed based on their special advantages and limitations by a person skilled in the art.

Drying can be suitably carried out in a tray dryer, vacuum oven, air oven, or using fluidized bed drier, spin flash dryer, flash dryer, and the like.

Drying can be carried out under reduced pressure until the content of residual solvent reduces to the desired amount such as an amount that is within the limits given in ICH guidelines. And drying temperatures and pressures are chosen based on the volatility of the solvent being used and the foregoing should be considered as only a general guidance. Detailed descriptions of the techniques and of the equipment used in drying processes are given in the literature.

Therefore, inventors needed to develop a new method to achieve acceptable residual solvent levels in active ingredient.

Inventors have encountered that the preparation of imatinib mesylate from IPA or THF results frequently in IPA or THF solvate forms of imatinib mesylate.

In aspect of quantity of residual solvent, obtained solvates contain significantly high content of IPA and THF. The residual content was determined by gas chromatography as following:
- between 12000 ppm and 15000 ppm for IPA
- between 5000 ppm and 9000 ppm for THF

The obtained IPA-DIPE and THF solvates by the process of the present invention, unless stated otherwise are characterized with the conditions described in following.

### Method for IPA and DIPE Detection:

Column Stationary phase: DB-Waxetr or equivalent)

Particle Size : 1.00 µm

Length: 50.0 m

Diameter: 0.32 mm

### Gas Parameters

Carrier gas: Nitrogen

Flow : 2,5 mL/min

Injection Port Temperature: 150 °C

Split ratio:1/10

Detector Temperature: 250 °C

Flow Control : Linear Velocity

Purge Flow: 3.0 ml/min

### Column

Starting Temperature:50°C

Equilibrium time: 3 min

**Table 1**

| Rate(°C/min) | Temperature( °C) | Hold Time (min) |
|---|---|---|
| - | 50 | 1 |
| 20 | 120 | 0 |
| 40 | 220 | 7 |

Makeup Gas: Nitrogen

Makeup Flow :30.0 mL /min

H₂ Flow : 40.0 mL /min

Air Flow : 450.0 mL/min

### Headspace parameters of Agilent GC

Oven Temperature: 90°C

Loop Temperature: 95°C

Transfer line Temperature: 100°C

Vial Equilibration: 15 min

Loop Fill :0.2 min

Loop Equilibration :0.5 min

Inject:1.00 min

Pressurization: 0.2 min

GC cycle : 21 min

Retention Times: DIPE: 3.1 min, IPA: 4.8 min

### Method for IPA and THF Detection:

Stationary phase: Restek Rtx®-1701 Cat. No. 12075

Length: 1 05 m

Diameter: 0.32 mm

Film thickness: 1.5 µm

### Gas flow

Flow Rate (He): 2.0 ml / min

Flow Rate (H₂): 40 ml / min

Flow Rate (Air): 400 ml / min

Make up flow: 30 ml / min

Purge Flow: 3 ml / min

Flow Control Mode: Linear Velocity

### Oven Temperature Program:

**Table 2**

| Ramp Rate (°C/min) | Column Temperature (°C) | Time (min) |
|---|---|---|
| 0 | 40 | 2 |
| 10 | 100 | 6 |
| 40 | 240 | 10 |

Injection Port Temperature: 165°C

Detector Temperature: 300°C, FID

Split Ratio: 1/10

### Headspace Parameters (for CombiPal)

Sample Volume: 1.0 ml

Incubation Temperature: 110°C

Incubation Time: 30 min

Agitator speed (rpm): 500

Agitator On time: 5 s

Agitator Off time: 2 s

Syringe Temperature: 115°C

Fill speed : 200 µL / s

Pull delay: 100 ms

Injection speed : 250 µL / s

Pre inject Delay : 100 ms

Post inject Delay : 100 ms

Syringe Flush: 1 min.

GC Run time: 35 min.

Retention Times: THF: 7.2 min, IPA: 8.9 min

The permissible limit of detection is 5000 ppm for IPA and 720 ppm for THF according to ICH of Technical Requirements for Registration of Pharmaceuticals for Residual Solvents, Q3C (R5) dated February 2012.

Although inventors have dried imatinib mesylate at 80 °C for 14 hours, the pharmaceutically accepted levels of residual solvent content for IPA and THF have not been reached. Nor vacuum drying at 105 °C for several hours has resulted in removal of isopropanol to more than 5000 ppm. The results of GC measurements showed that only temperature above 180 °C yields imatinib mesylate crystalline solid free of residual isopropanol. However, elevated temperatures are not recommended in pharmaceutical processes due to the possibility of degradation of active substance. Also, heating at high temperatures and long period of time is not desirable in economical aspect.

None of the above mentioned techniques is completely satisfactory because they all require an additional process such as extended period of drying time and energy consuming processes to remove trace of solvent.

Inventors needed to develop a new method to achieve acceptable residual solvent levels in active pharmaceutical ingredient.

To solve the technical problems explained foregoing, our inventors have developed a desolvation process which includes recrystallization of solvate forms of imatinib from a suitable solvent mixture containing seed crystals of imatinib for seeding and then drying procedure. Imatinib mesylate is produced by desolvation of IPA or THF solvate forms of imatinib after recrystallized these said solvate forms in a mixture of diisopropyl ether : isopropanol (DIPE: IPA) and then drying of formed crystals by heating under vacuum at an appropriate temperature for a time sufficient to obtain the desired reduction in solvent content.

Solvate forms of imatinib mesylate with IPA and THF have been identified with Scanning Electron Microscope (SEM-JCM -5000 NeoScope model). SEM images.

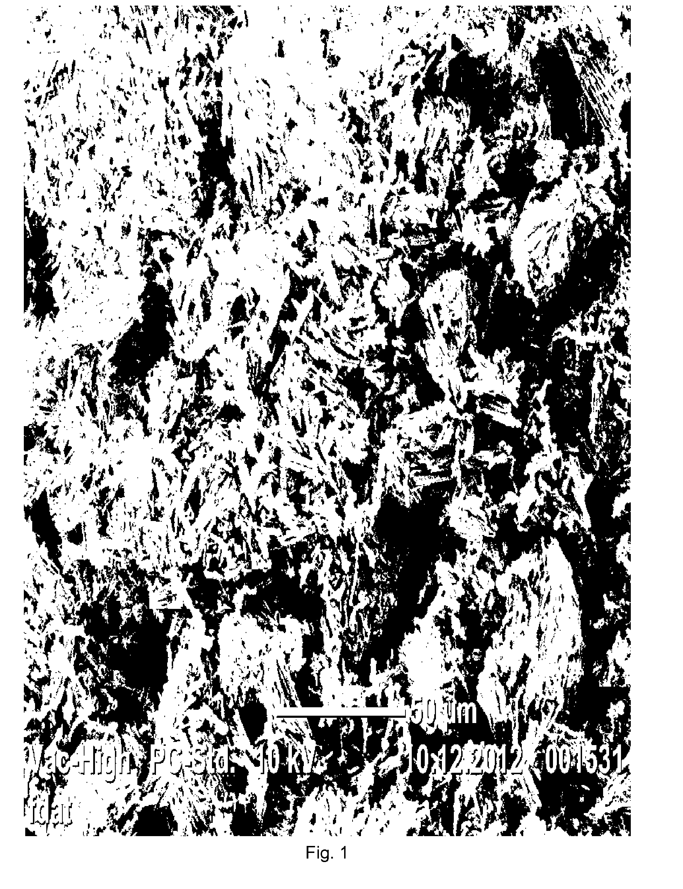

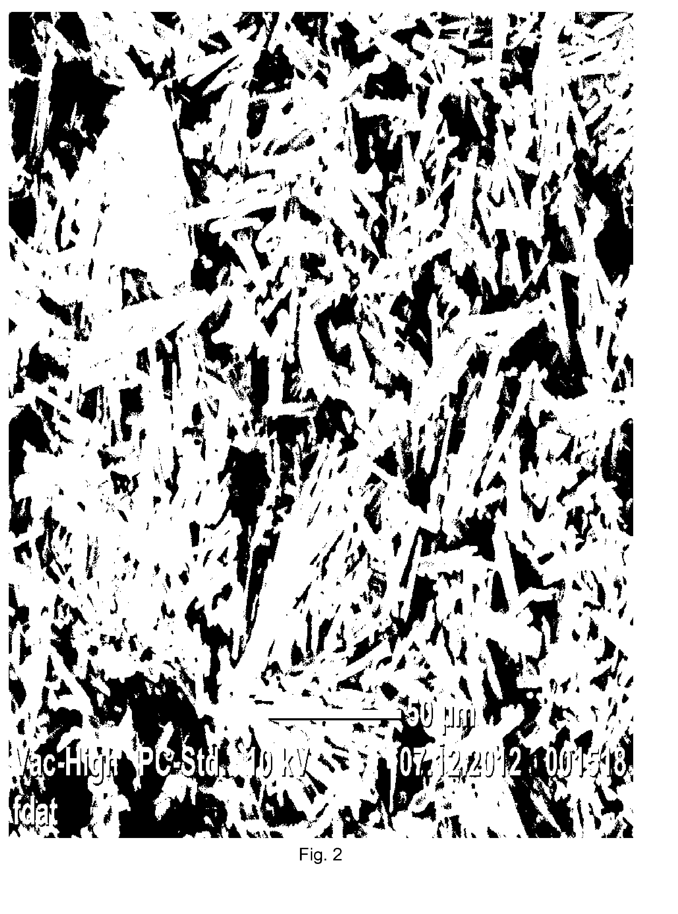

According to Fig. 1 and Fig. 2, it can be seen that IPA or THF solvent molecules solvate the crystals of imatinib mesylate by reducing the distance among the crystals but not change the crystal form of imatinib.

And the crystalline alpha form of imatinib mesylate which is characterized by needle shaped crystals does not change upon solvation with IPA or THF.

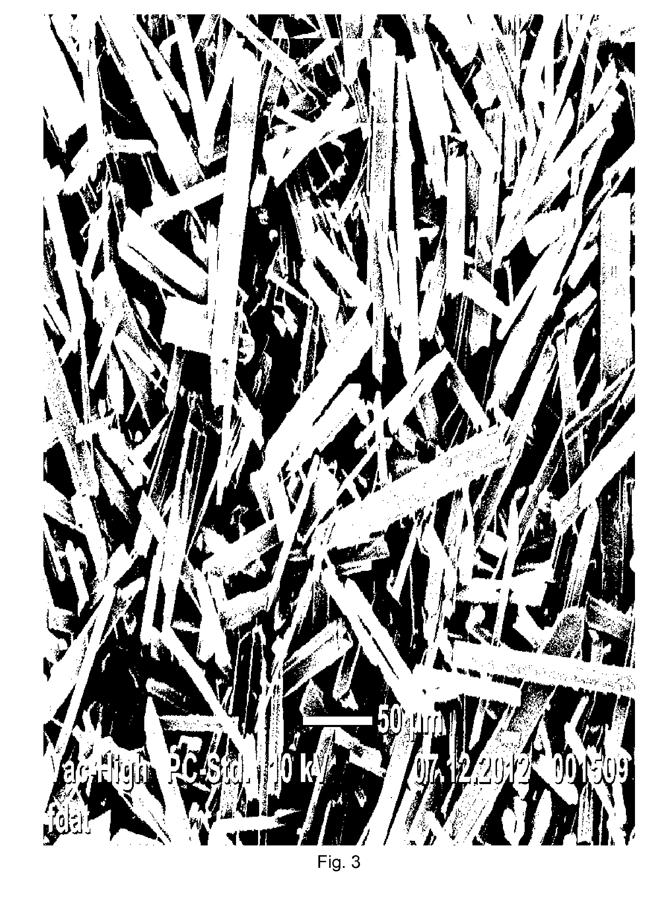

When Fig. 1 or Fig. 2 with Fig. 3.of alpha imatinib crystals are compared, are observed as stacked, Fig.3 show the crystals of α-crystal form of imatinib mesylate after desolvation process which includes recrystallization of solvate forms of imatinib from a suitable solvent mixture containing seed crystals of imatinib for seeding and then drying procedure.

To show efficiency of this developed technique, the results are shown as follows.

Table 1 shows imatinib Mesylate-IPA solvate IPA's content before and after recrystallization of IPA- imatinib mesylate solvate from mixture of DIPE: IPA under vacuum drying conditions.

**Table 3**

| Sample No | Drying Temperature (°C) | Drying Time (h) | Content of Residual IPA*(ppm) | Content of Residual IPA**(ppm) |
|---|---|---|---|---|
| 1 | 65 | 12 | 15610 | 4900 |
| 2 | 85 | 8 | 13200 | 4150 |
| 3 | 105 | 8 | 13000 | 4120 |
| 4 | 85 | 6 | 14300 | 4200 |

| | | | | |
|---|---|---|---|---|
| *Before recrystallization of IPA-imatinib mesylate solvate from mixture of DIPE: IPA **After recrystallization of IPA-imatinib mesylate solvate from mixture of DIPE: IPA | | | | |

The amount of residual solvent has been determined by gas chromatography equipped with FID detector.

According to Table 1, when imatinib mesylate- IPA solvate form is heated at 85°C under vacuum for 8 hours the amount of residual solvent has been determined as 13200 ppm for IPA.

The results shown in Table 1 reveal that the quantity of IPA is lower when imatinib mesylate IPA solvate form is recrystallized from mixture of DIPE: IPA in comparison to process without recrystallization step.

Table 1 shows imatinib mesylate-THF solvate THF's content before and after recrystallization of THF- imatinib mesylate solvate from mixture of DIPE: IPA under vacuum drying conditions.

**Table 4**

| Sample No | Drying Temperature (°C) | Drying Time (h) | Content of Residual THF *(ppm) | Content of Residual THF**(ppm) |
|---|---|---|---|---|
| 1 | 65 | 12 | 7784 | 450 |
| 2 | 85 | 8 | 7550 | 325 |
| 3 | 105 | 8 | 7280 | 320 |

| | | | | |
|---|---|---|---|---|
| *Before recrystallization of THF- imatinib mesylate solvate from mixture of DIPE: IPA. **After recrystallization of THF- imatinib mesylate solvate from mixture of DIPE: IPA. | | | | |

According to Table 2, when imatinib mesylate- THF solvate form is heated at 85 °C under vacuum for 8 hours, the amount of residual solvent has been determined by GC as 7550 ppm for THF.

The results shown in Table 2 reveal that the quantity of THF is lower when imatinib mesylate THF solvate form is recrystallized from mixture of DIPE: IPA in comparison with process without recrystallization step.

In another aspect of present invention, the process for the preparation of imatinib mesylate comprises steps of:
(a) Dissolving of imatinib mesylate solvate or mixture of crystalline forms in a suitable solvent or mixture of solvent.
(b)Heating the solution in step (a) at a suitable temperature until to get clear solution.
(c)Adding of seeds of imatinib mesylate crystalline form α into another mixture of solvent.
(d)Stirring the solution at a suitable temperature.
(e)Adding the solution in step (b) into the second solution in step (d)
(f)Stirring the mixture of solution in step (e) at a suitable temperature for a suitable time.
(g)Cooling the solution in step (f) to precipitate the solid.
(f)Drying formed crystals of imatinib mesylate under vacuum for a suitable time.

In another aspect of the invention, all above steps (a to f) are individually described herein as disclosure of the present invention.

The solvate forms of imatinib mesylate in step (a) are IPA and THF solvate forms. The mixture of crystalline forms in step (a) are composed of alpha and β crystalline forms of imatinib mesylate.

The solution of IPA or THF solvate form of imatinib mesylate may be obtained by dissolving IPA or THF solvate forms of imatinib mesylate in a suitable mixture of solvent.

Crystallization in step (c) is carried out by using DIPE: IPA in the range of 1:4 to 4:1 by volume.

Suitable mixture of solvent in step (a) is IPA: water and DIPE: IPA in step(c).

Suitable temperature are as follows;
- from about 25 °C to 85 °C in step (b) for dissolution can ranged, preferably 80 °C.
- from about 40 °C to 55 °C-in step (e) for stirring mixture of solution can be ranged, preferably about 50 °C.

The time period for stirring the solution in step (f) may be in the range from about 30 minutes to about 2 hours, or longer hours.

Optional drying in step (h) may be carried out in conventional equipments, which may be selected from tray dryer, vacuum oven, air oven, fluidized bed drier, spin flash dryer, flash dryer and the like. The drying temperatures for carrying out the drying of the crystalline α form of imatinib mesylate may range from 40 °C to about 110 °C with vacuum. The drying may be carried out for any desired time until the desired product quality parameters are achieved. Preferably, time periods may range from about 1 to 14 hours.

In another aspect of this invention, drying efficiency has increased by this method. For example; for direct drying IPA solvated imatinib mesylate at 105 °C for 8 hours, the amount of IPA is determined as 13000 ppm (above the permissible limit is below 5000 ppm according to ICH).

On the other hand, by the proposed method of desolvation of IPA solvated imatinib mesylate by recrystallization and applying drying process, the amount of IPA is determined as 4200 ppm (below the acceptable limit for IPA). The same procedure is applied for THF solvated imatinib mesylate. The amount of THF is determined as 7280 ppm (permissible limit below 720 ppm) for direct drying THF solvated imatinib mesylate at 105 °C for 8 hours. After desolvation of imatinib solvated with THF following drying at same temperature for same drying time, the amount of THF reduced to below 350 ppm, respectively 332 ppm.

So, the presented invention is quite satisfactory in economic terms in respect of consuming less time and energy.

As a result of recrystallization step of imatinib IPA or THF solvate forms, desolvation of said solvate forms occurs and the drying time is shortened to get rid of residual solvent.

The following examples are provided solely for purposes of illustration, and should not be construed as limiting the scope of the application in any matter.

### Example: Preparation of Imatinib mesylate alpha form from Imatinib mesylate THF solvate:

1.10 g of Imatinib mesylate in crystalline form-α is dissolved in a mixture of IPA: Water (40:5) at 75-80 °C to get clear solution in a reactor. Another reactor is charged with 100 ml of IPA and 25 ml of DIPE and heated to about 55-60 °C followed by the addition of seeds of imatinib mesylate crystalline form α (5% w/w). The solution in the first reactor is added slowly into the second reactor. The reaction mixture is stirred at 50 °C for 1 hour. Then the solution is cooled to 30 °C slowly, to obtain imatinib mesylate alpha crystals form. The formed crystals are filtered and dried under vacuum for about 4 h 80 °C.9 g of alpha form of imatinib mesylate is obtained and identified through GC.

### Example: Preparation of Imatinib mesylate alpha form from Imatinib mesylate IPA solvate

Imatinib mesylate in crystalline form-α is dissolved in a mixture of IPA: Water (40:5.5) at 75-80 °C to get clear solution in a reactor.

Another reactor is charged with 110 ml of IPA and 25 ml of DIPE and heated to about 55-60°C followed by the addition of seeds of (3% w/w) imatinib mesylate crystalline form alpha.

The solution prepared in the first reactor is added slowly into the second reactor. The reaction mixture is stirred at 50 °C for 1 hour. Then the solution is cooled to 30 °C slowly, to obtain crystals of imatinib mesylate in α form. The formed crystals are filtered and dried under vacuum. Drying may be carried out for any desired time until the desired product purity is achieved, such as time periods from 1 to 20 hours, or longer. The solid obtained is dried at temperature about 80°C, under vacuum for a period about 4 to 14 hours.

9.2 g of alpha form of imatinib mesylate is obtained and identified through GC.

### Example: Preparation of Imatinib mesylate form alpha from Imatinib mesylate alpha and beta mixture:

10 g of imatinib mesylate as a mixture of α and β is dissolved in a mixture of IPA: Water (40:6) at 75-80°C to get clear solution in a reactor.

Another reactor is charged with 120 ml of IPA and 30 ml of DIPE and heated to about 55-60 °C followed by the addition of seeds of imatinib mesylate crystalline form-α (5% w/w).

The solution in the first reactor is added slowly into the second reactor. The reaction mixture is stirred at 50 °C for 1 hour. Then the solution is cooled to 30 °C slowly, to obtain imatinib mesylate alpha crystals form. The formed crystals are filtered and dried under vacuum for about 14 h in 80 °C.

9.5 g of α form of imatinib mesylate is obtained and identified through GC.

## Claims

1. A process for preparing crystalline imatinib mesylate in pure alpha form by desolvation of imatinib mesylate solvate forms, the process comprising: (a) dissolving alpha crystalline or alpha and beta crystalline mixture of imatinib mesylate solvate in a mixture of isopropyl alcohol and water at a suitable temperature; (b)providing an another solution containing seeds of imatinib mesylate crystalline form alpha in a mixture of diisopropyl ether and isopropyl alcohol; (c)adding solution in step (a) into the solution in step (b); (d) allowing the solution mixture in step (c) to cool for precipitation; (e) filtering to obtain the imatinib mesylate crystal alpha form, and then (f) drying formed pure alpha form imatinib mesylate crystals for a time residual solvent content is acceptable.

2. According to claim 1, added seed crystals in step (a) are in an amount of about 5 weight % relative to α-form imatinib mesylate.

3. According to claim 1, solvates form of imatinib mesylate is selected from the group consisting tetrahydrofuran solvate, dioxane solvate, ethanol solvate and isopropyl alcohol solvate.

4. According to claim 1, the drying to obtain pure alpha form imatinib mesylate crystals can be carried out in a tray dryer, vacuum oven, air oven, or fluidized bed dryer, spin flash dryer, flash dryer.

5. According to claim 1, the temperature for carrying out the drying of the crystalline α form of imatinib mesylate may range from 40 °C to about 110 °C with vacuum until residual solvent content is acceptable.

6. According to claim 1, the residual solvent content after drying step is below 750 ppm for THF.

7. According to claim 1, the residual solvent content after drying is below 5000 ppm for isopropyl alcohol.

8. According to claim 1, the dried α-form crystals imatinib mesylate are obtained in a yield greater than about 90%.
